# EUROPÄISCHE PATENTANMELDUNG

(11) **EP 1 728 520 A1**
(43) Veröffentlichungstag der Anmeldung: **06.12.2006**
(21) Anmeldenummer: 05009822.7
(22) Anmeldetag: 04.05.2005
(51) Int. Cl.: A61K 51/04

(54) **Verwendung von spezifisch an das Serotonin-Transport-Protein (SERT) bindenden Radiopharmaka zur Herstellung eines Diagnostikums zur Untersuchung von Kopfschmerzerkrankungen**

(71) Anmelder: Charité - Universitätsmedizin Berlin, 10117 Berlin (DE)
(72) Erfinder: Schuh-Hofer, Sigrid, 10961 Berlin (DE); Guy, Arnold, 71065 Sindelfingen (DE); Richter, Matthias, 13055 Berlin (DE)
(74) Vertreter: Krauss, Jan

(57) **Zusammenfassung**

Die vorliegende Erfindung betrifft die Verwendung von spezifisch an das Serotonin-Transport-Protein (SERT) bindenden Radiopharmaka zur Herstellung eines Diagnostikums zur Untersuchung von Kopfschmerzerkrankungen. Bei den Radiopharmaka handelt es sich um Tracer, die die Eigenschaft haben, im Rahmen einer Untersuchung mit Positron-Emission-Tomography (PET) oder Single Photon Emission Tomography (SPECT) spezifisch an das Serotonin-Transport-Protein zu binden, um damit die Verfügbarkeit des Serotonin-Transport-Proteins im Gehirn von Kopfschmerzpatienten nachzuweisen. Die vorliegende Erfindung betrifft weiterhin ein Verfahren zum Screening auf Substanzen, die selektiv die Bindung dieser spezifischen Radiopharmakaan den Serotonintransporter modifizieren. Diese Substanzen stellen z.B. Vorstufen putativer Therapeutika zur Behandlung von Kopfschmerzerkrankungen dar.

## Beschreibung

### Inhaltverzeichnis

| | | Seite |
|---|---|---|
| 1. | Einleitung | 3 |
| 1.1 | Beschreibung des wissenschaftlichen Problems | 3 |
| 2. | Einsatz der Erfindung | 12 |
| 2.1 | Diagnose der Migräne und differentialdiagnostische Abgrenzung der Migräne | 13 |
| 2.2 | Differentialdiagnose des Clusterkopfschmerzes | 13 |
| 2.3 | Pathophysiologie des arzneimittelinduzierten Kopfschmerzes | 13 |
| 2.4 | Verlaufsuntersuchungen mittels spezifisch an den SERT bindenden Radiopharmaka und -SPECT unter medikamentöser Migränetherapie | 14 |
| 3. | Beispiele | 14 |
| 3.1 | Verwendetes Radiopharmakon | 15 |
| 3.2 | Patienten/Probanden | 15 |
| | 3.2.1 Einschlusskriterien | 16 |
| | 3.2.2 Ausschlusskriterien für Patienten | 16 |
| | 3.2.3 Abbruchkriterien | 17 |
| | 3.2.4 Unerlaubte Medikamente (bekannte Interaktion mit ¹²³I-ADAM): | 17 |
| 3.3 | Projektbedingte Untersuchungen | 18 |
| 3.4. | Untersuchungsvorgang | 18 |
| 3.5 | Zielregion | 19 |
| 3.6 | Auswertung der Daten: | 19 |
| 3.7 | Zielregion (Pontomesencephale Region) - Referenzregion (Okzipitallappen)/Referenzregion | 20 |
| 3.8 | Ergebnisse | 20 |
| 4. | Literatur | 22 |
| 5. | Patentansprüche | 28 |
| 6. | Zusammenfassung | 30 |

### 1. Einleitung

Die vorliegende Erfindung betrifft die Verwendung von spezifisch an das Serotonin-Transport-Protein (SERT) bindenden Radiopharmaka zur Herstellung eines Diagnostikums zur Untersuchung von Kopfschmerzerkrankungen. Bei den Radiopharmaka handelt es sich um Tracer, die die Eigenschaft haben, im Rahmen einer Untersuchung mit Positron-Emission-Tomography (PET) oder Single Photon Emission Tomography (SPECT) spezifisch an das Serotonin-Transport-Protein zu binden, um damit die Verfügbarkeit des Serotonin-Transport-Proteins im Gehirn von Kopfschmerzpatienten nachzuweisen. Die vorliegende Erfindung betrifft weiterhin ein Verfahren zum Screening auf Substanzen, die selektiv die Bindung dieser spezifischen Radiopharmaka an den Serotonintransporter modifizieren. Diese Substanzen stellen z.B. Vorstufen putativer Therapeutika zur Behandlung von Kopfschmerzerkrankungen dar.

### 1.1 Beschreibung des wissenschaftlichen Problems

Das biogene Amin Serotonin ist im zentralen Nervensystem nicht nur wesentlich bei der Steuerung kognitiver und emotionaler Prozesse sowie bei der Regulation des Schlaf-Wach-Rhythmus beteiligt, es spielt u.a. auch eine wichtige schmerzmodulatorische Rolle im Rahmen der nociceptiven Transmission.

Bereits seit Jahrzehnten wird eine Beteiligung des serotonergen Systems in der Pathophysiologie von Kopfschmerzerkrankungen postuliert. Insbesondere in Hinblick auf die Migräne und den Clusterkopfschmerz gibt es hierfür zahlreiche indirekte Hinweise (s.u.) (Ferrari & Saxena 1993).

Die Migräne gehört mit einer Prävalenz von 12- 18% in der weiblichen Bevölkerung und ca. 5-8% in der männlichen Bevölkerung zu den - auch ökonomisch hochrelevanten - Volkskrankheiten (Rassmussen, 1993, Russell et al., 1995). Im Gegensatz dazu handelt es sich beim Clusterkopfschmerz um eine eher seltene primäre Kopfschmerzerkrankung (Bahra et al. 2002). Ungeachtet des erheblichen individuellen Leidensdrucks betroffener Patienten stellt insbesondere die Migräne aufgrund hoher ambulanter Therapiekosten sowie hoher indirekter Kosten durch Arbeitsausfälle eine enorme sozioökonomische Belastung dar (Davies GM et al, 1999). Beide primäre Kopfschmerzerkrankungen sind durch das Auftreten heftiger, unilateraler Schmerzattacken sowie durch jeweils spezifische autonome Begleiterscheinungen gekennzeichnet. Migräneattacken treten teils lebenslang mit individuell unterschiedlicher Attackenfrequenz auf. Im Gegensatz dazu handelt es sich beim Clusterkopfschmerz in bis zu 90% der Fälle um eine episodische Erkrankung mit einer durchschnittlichen Episodendauer von ca. 8 Wochen.

Die Hypothese von einer Rolle des Serotonin in der Pathophysiologie von Migräne und Clusterkopfschmerz beruhte bis vor kurzem vor allem auf *indirekten* Hinweisen: Zur bedeutendsten *empirischen* Evidenz gehört dabei die Tatsache, dass serotonerge Substanzen mit Erfolg in der Therapie von Migräne- und Clusterkopfschmerz eingesetzt werden. Neben dem Einsatz von unselektiven Serotonin-Agonisten, den sogenannten "Ergotaminen" (Jost und Selbach, 2001) haben sich seit Anfang der 1990er Jahre vor allem die hochselektiven Serotonin-Rezeptoragonisten, die am 5HT1_{B} und am 5HT1_{D}-Rezeptor-Subtyp wirken, in der Akuttherapie der Migräne, aber auch des Clusterkopfschmerzes etabliert (Diener & Eikermann, 2003, Ferrai et al. 2001; Ekbom et al., 1995; Schuh-Hofer et al., 2002). Serotonerge Substanzen sind nicht nur in der Akuttherapie, sondern auch in der Prophylaxe von Migräne und Clusterkopfschmerz wirksam: So zählt beispielsweise Methysergid, ein 5HT-2-Rezeptorantagonist mit agonistischer Wirkung an 5HT1B/1D-Rezeptoren, zu den potentesten Prophylaktika in Migräne und Clusterkopfschmerz überhaupt (Silberstein SD, 1998). Vor allem in den USA gehören darüber hinaus auch trizyklische Antidepressiva zu den Mitteln der 1. Wahl in der Migräneprophylaxe (Silberstein SD, 2000).

Aufgrund technischer Limitation ist es bis vor kurzem nicht möglich gewesen, das serotonerge System im menschlichen Gehirn "in vivo" darzustellen. Untersuchungen zum Serotoninstoffwechsel von Kopfschmerzpatienten mussten sich daher auf die Bestimmung von Serotonin-Stoffwechselprodukten in Blut, Thrombozyten oder Urin von Patienten beschränken. Solche Untersuchungen wurden vor allem bei Migränepatienten, aber auch bei Patienten mit arzneimittelinduziertem Kopfschmerzen (Srikiatkhachorn et al., 1996) durchgeführt. Trotz zahlreicher Untersuchungen waren die Ergebnisse heterogen oder sogar widersprüchlich, was teils auf methodische Probleme zurückgeführt werden kann (Übersicht bei Ferrari 1993, d'Andrea 1995).

Nach der Identifikation des Gens, welches für das Serotonin-Transport-Protein codiert (5-HTT-Gen) (Ramamoorthy et al., 1993), wurden die bisher bekannten Polymorphismen des 5-HTT-Gens auf ihre Bedeutung für die Migränepathophysiologie untersucht. In den bisherigen genetischen Untersuchungen konnte jedoch noch keine überzeugende Evidenz dafür gefunden werden, dass allelische Variationen im SERT- -Gen zu einer erhöhten Suszeptibilität für Migräne führen (Ogilvie et al. 1998, Yilmaz et al., 2001; Kotani et al., 2002, Juhasz et al., 2003). Somit ist derzeit unklar, ob oder inwieweit genetische Veränderungen im SERT-Gen in die Migränepathophysiologie involviert sind.

Neue indirekte Hinweise für eine Rolle des serotonergen Systems in der Migränepathophysiologie ergaben sich aus einer bildgebenden Untersuchung von Weiller et al. In einer [¹⁵O]H₂O-PET-Untersuchung konnte dabei sowohl während akuter Migräneattacken als auch nach pharmakologischer Beendigung der Attacken eine deutliche regionale Blutflussteigerung im Bereich des Hirnstamms von Migränepatienten beobachtet werden (Weiller et al. 1995). Wenngleich aufgrund der limitierten räumlichen Auflösung eine anatomische Zuordnung zu einzelnen Kerngebieten im Hirnstamm nicht möglich war, umfasste die Region mit dem erhöhten Blutfluß anatomisch einen Kernkomplex im Hirnstamm, der als nahezu ausschließliches Ursprungsgebiet für serotonerge Neurone im Gehirn angesehen wird. Bei diesen Kernen handelt es sich um die sogenannten "Raphe-Kerne", die im Hirnstamm in zwei Gruppen "übereinander" angeordnet sind. Die weiter rostral, in Mesencephalon und Pons gelegenen Raphe-Kerne (rostraler Raphe-Kernkomplex), projizieren in Richtung Großhirn und Zwischenhim, während die in der Medulla oblongata gelegenen Raphe-Kerne (kaudaler Raphe-Kernkomplex) im wesentlichen zum Rückenmark projizieren (Baumgarten & Göthert 2000). Die Raphe-Kerne spielen nach bisherigen Erkenntnissen eine ganz entscheidende Rolle bei der *Schmerzmodulation* (Andersen & Dafny 1983, Dong et al., 1991, Prieto-Gomez et al. 1989). Zusammengefasst deuteten die von Weiller durchgeführten Blutfluß-PET-Untersuchungen im Gehirn von Migränepatienten indirekt auf eine Aktivierung im Bereich der Raphe-Kerne hin, die eine wesentliche Rolle in der Nocifension spielen. Dennoch blieb das Untersuchungsergebnis von Weiller insofern unspezifisch, als die Methode lediglich die Darstellung von Blutflussänderungen, nicht jedoch die Darstellung des serotonergen Stoffwechsels ermöglicht.

Die erste direkte bildgebende Pilotuntersuchung zum Serotoninstoffwechsel von Migränepatienten wurde 1999 von Chugani durchgeführt. Chugani wählte dabei eine Methode, bei der die cerebrale Kapazität der Serotoninsynthese mittels PET- und dem Radiopharmakon ¹¹C-α-methyl-L-tryptophan ermittelt werden kann (Chugani et al., 1999). In dieser an 11 Patienten durchgeführten Untersuchung fand sich bei 8 Patienten mit Migräne ohne Aura eine global erhöhte Kapazität für die Serotoninsynthese. Lokale Unterschiede in anatomischen Regionen, die in der Migränepathophysiologie eine hervorgehobene Rolle spielen, fanden sich in dieser Untersuchung jedoch nicht. Aufgrund der geringen Patientenzahl handelte es sich hier um eine Pilotstudie.

Die bisher unzureichenden wissenschaftlichen Belege für eine Störung des serotonergen Systems im Gehirn von Kopfschmerzpatienten waren der Anlaß der Erfinder, mittels neuer bildgebender Verfahren die Hypothese eines gestörten Serotoninstoffwechsels bei Kopfschmerzpatienten zu untersuchen. Ihre Untersuchungsreihe konzentrierte sich dabei auf Migränepatienten. Erst seit kurzem ist es möglich, mit nuklearmedizinischen Methoden (SPECT und PET) das Serotonin-Transport-Protein in vivo darzustellen (Brücke et al. 1993, Okada et al. 1998, Hesse et al., 2004). Das erste hierzu eingesetzte Radiopharmakon war das Tropanderivat Beta-CIT (Hill et al, 1998). Nachteil dieses Radiopharmakons ist die relativ geringe serotonerge Spezifität, da neben den Serotonintransportern (SERT) auch die Dopamintransporter (DAT) radioaktiv markiert werden und eine exakte Beurteilung des zentralen Serotoninsystems nur eingeschränkt möglich ist (Bergstrom et al. 1994, 1997, Pirker et al. 1995, Hiltunen et al. 1998, Tauscher et al. 1999, Jones et al., 1998). In jüngster Vergangenheit wurden 3 neue Radiopharmaka zur Darstellung serotonerger Transportersysteme mit SPECT entwickelt und vorgestellt: ¹²³I-IDAM, ¹²³I-ODAM (Zhuang et al. 2000) und ¹²³IADAM (Oya et al. 2000). Alle drei Radiopharmaka zeichnen sich durch eine hohe Selektivität (Kung et al. 1999, Choi et al. 2000) und ihre hervorragende Eignung für die Bildgebung mit SPECT (Acton et al. 1999) aus. Vergleichende Untersuchungen deuten jedoch darauf hin, dass ¹²³IADAM aufgrund seines höheren Uptakes, seiner höheren Selektivität und seiner - basierend auf einer niedrigeren unspezifischen Bindung - besseren Bildqualität den anderen beiden Radiopharmaka überlegen ist (Acton et al. 2001). Größter Vorteil gegenüber dem bislang verwendeten Tropanderivat ß-CIT ist seine 10.000fach höhere Affinität zum Serotonintransporter verglichen mit dem Dopamin- oder Norepinephrintransporter (Oya et al 2000). Durch ^{I23}I-ADAM-SPECT ist es möglich, den Serotonin-Transporter in vivo hochselektiv darzustellen und semiquantitativ zu beurteilen und damit Störungen zu untersuchen, bei denen bislang nur indirekte Hinweise auf eine pathogenetische Beteiligung des serotonergen Systems vorliegen. Abgesehen von den SPECT-Radiopharmaka gelten derzeit auch die PET-Radiopharmaka ¹¹C-McN 5652, (S)-([¹⁸F]fluoromethyl)-(+)-McN5652 und ¹¹C-DASB als sehr spezifisch und gut geeignet, um das Serotonin-Transport-Protein in vivo darzustellen.

Wie weiter oben bereits ausgeführt, beruhen die Diagnosen "Migräne" und "Clusterkopfsschmerz" derzeit lediglich auf einer ausführlichen Kopfschmerzanamnese. Die Internationale Kopfschmerzgesellschaft hat standardisierte Diagnosekriterien für die beiden Kopfschmerzerkrankungen erarbeitet, die erst kürzlich in einer revidierten Fassung veröffentlicht wurden (Cephalalgia 2004, 24: Suppl 1). Zum heutigen Zeitpunkt stehen weltweit - abgesehen von diesen anamnestischen Kriterien - keinerlei diagnostischen Verfahren zur Verfügung, um bei Kopfschmerzpatienten die Diagnose einer Migräne sicher zu stellen. Dabei hat sich im Rahmen langjähriger klinischer Erfahrung in einem hochspezialisierten Kopfschmerzzentrum gezeigt, dass vielen Ärzten im ambulanten Versorgungsbereich die Diagnose von Migräne und Clusterkopfschmerz sowie deren Differentialdiagnosen oft erhebliche Probleme bereitet. Wenngleich epidemiologische Studien hierzu fehlen, muss von einer hohen Zahl von Fehldiagnosen ausgegangen werden. Dies wirkt sich - aufgrund unzureichender oder falscher Therapieansätze- letztlich nicht nur auf den einzelnen Patienten aus, sondern belastet das Gesundheitssystem auch aus ökonomischer Sicht. Gerade unter diesem Aspekt ergibt sich eine wesentliche Anwendungsmöglichkeit für die Verwendung von SERT-Radiopharmaka zur Diagnose und Differentialdiagnose der Migräne, zumal die Migräne z.B. neben dem Diabetes mellitus oder dem Rückenschmerz zu einer der großen Volkskrankheiten gehört.

Wie oben ausgeführt, gibt es Vermutungen, nach denen auch beim Clusterkopfschmerz das serotonerge System eine pathophysiologische Rolle spielt. In [¹⁵O]H₂O-PET-Untersuchungen an Clusterkopfschmerzpatienten konnte eine spezifische, regionale Blutfflussteigerung in ipsilateralen Hypothalamuskerngebieten während akuter Clusterkopfschmerzattacken nachgewiesen werden (May A et al. 1998). Der Hypothalamus gehört zu einem wesentlichen Projektionsgebiet serotonerger Raphe-Kerne. Chronobiologische Aspekte wie das circadiane und circanuelle Auftreten von Clusterkopfschmerzattacken, die für die Pathophysiologie des Clusterkopfschmerzes eine wichtige Rolle spielen, wären mit einer serotonergen Dysfunktion im Bereich des Hypothalamus (insbesondere im Bereich des Nucleus suprachiasmaticus) erklärbar (Glass JD, 2003). Daher ist es denkbar, dass sich bei Clusterpatienten - im Gegensatz zu Migränepatienten - eine spezifische regionale Dysregulation des Serotonin-Transportproteins im Bereich des Hypothalamus findet. Hiermit würde sich also die Anwendungsmöglichkeit von spezifischen SERT-Radiopharmaka auf die Differentialdiagnose des Clusterkopfschmerzes erweitern.

Neben den oben genannten "primären" Kopfschmerzerkrankungen Migräne und Clusterkopfschmerz gibt es auch für die häufigste "sekundäre" Kopfschmerzerkrankung, den arzneimittelinduzierten Kopfschmerz, Hinweise auf eine pathophysiologische Beteiligung des serotonergen Systems. Die Prävalenz des arzneimittelinduzierten Kopfschmerzes liegt bei ~ 1 % in der Bevölkerung (Castillo J et al., 1999). Der arzneimittelinduzierte Kopfschmerz entwikkelt sich auf dem Boden eines langjährigen Schmerzmittelabusus und führt klinisch zu einem Dauerkopfschmerz. In einer Studie zur Serotonin-Konzentration in Thrombozyten von Patienten mit arzneimittelinduziertem Kopfschmerz zeigte sich eine im Vergleich zu Migränepatienten signifikante Erniedrigung der Serotonin-Konzentration (Srikiatkhachorn & Anthony, 1996). Aufgrund der herausragenden Rolle von Serotonin in der Schmerzpathophysiologie ist daher ein ausgeprägter Serotoninmangel als Grundlage für den arzneimittelinduzierten Kopfschmerz möglich.
Es ist daher eine Aufgabe der vorliegenden Erfindung, ein Verfahren und entsprechende Komponenten dazu zur Verfügung zu stellen, mit dem eine Dysregulation des Serotonin-Transportproteins bei Patienten mit analgetika-induziertem Kopfschmerz zu überprüfen ist. Eine weitere Aufgabe der vorliegenden Erfindung betrifft dann auch die Diagnostik und Verlaufskontrolle von Patienten mit Schmerzmittelmissbrauch und die Verwendung im Rahmen der Medikamentenentwicklung.

Gemäß eines ersten Aspekts der vorliegenden Erfindung wird eine Aufgabe der vorliegenden Erfindung durch die Verwendung von spezifisch an das Serotonin-Transport-Protein (SERT) bindenden Radiopharmaka zur Herstellung eines Diagnostikums zur Untersuchung von Kopfschmerzpatienten gelöst. Besonders bevorzugt ist das ausgewählte Radiopharmakon ¹²³I-ADAM für die Hirn-SPECT-Szintigraphie.

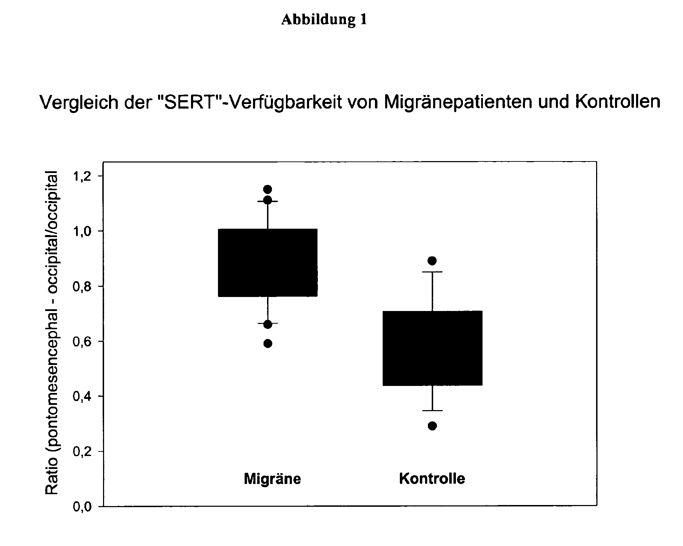

Es ist den Erfindern gelungen, durch den Einsatz der ¹²³I-ADAM- SPECT bei Migränepatienten eine deutliche Erhöhung der Verfügbarkeit des Serotonin-Transport-Proteins ("SERT") in der pontomesencephalen Region, der anatomischen Region des rostralen Raphe-Kemkomplexes, über eine erhöhte Radioaktivitätsspeicherung nachzuweisen (siehe Abbildung 1). Das Serotonin-Transport-Protein hat die Aufgabe, das biogene Amin Serotonin aus dem synaptischen Spalt in das Neuron zurückzutransportieren und regelt damit die Konzentration von im synaptischen Spalt verfügbarem Serotonin. Migränepatienten unterscheiden sich hinsichtlich der Verfügbarkeit dieses Serotonin-Transport-Proteins anhand der Untersuchungen im Rahmen der Erfindung in signifikanter Weise von Kontrollpersonen, die nicht unter einer Migräne leiden. Die hier dargestellten Ergebnisse sind die weltweit ersten Untersuchungsergebnisse zur Darstellung der Verfügbarkeit des Serotonin-Transport-Proteins im Gehirn von Migränepatienten. Nach Literaturrecherche sind die Erfinder die Ersten, die ein Radiopharmakon (¹²³I-ADAM), welches spezifisch an den SERT bindet, für die Darstellung des Serotonintransporters im Gehirn von Migränepatienten eingesetzt haben. Auch wenn nur ein Radiopharmakon, welches spezifisch an den SERT bindet (¹²¹I-ADAM), für diese Untersuchung eingesetzt wurde, ist aus wissenschaftlicher Sicht davon auszugehen, dass sich die Ergebnisse mit jedem Radiopharmakon, welches spezifisch am Serotonintransporter bindet, reproduzieren lassen. Im vorliegenden Fall (siehe Abbildung 1) wurde im Rahmen der vorliegenden Erfindung eine Erhöhung des Verhältnisses des SERT bei Migränepatienten von pontomesencephal zu occipital zwischen etwa 0,8 bis 1,0 und insbesondere 0,85 festgestellt. Entsprechende Veränderungen der Verhältnisse werden auch in anderen Kopfschmerzformen und mit anderen verwendeten Radiopharmaka und Untersuchungsverfahren erwartet. Der Fachmann kann somit auf Basis der hier zur Verfügung gestellten speziellen Lehre ohne weiteres andere statistisch relevante Verhältnisse und deren Verschiebungen ermitteln, die je nach Schmerz, verwendetem Radiopharmakon, Zahl der Probanden und Untersuchungsverfahren zahlenmäßig unterschiedlich ausfallen werden. Dies ist jedoch vom allgemeinen Konzept der vorliegenden Erfindung umfaßt.

Die von den Erfindern entwickelte Methode könnte in Zukunft Eingang in die klinische Routine im Sinne eines diagnostischen, nicht-invasiven Tools, gewinnen.

Erfindungsgemäß kann ein bevorzugtes Radiopharmakon ein herkömmliches PET-Radiopharmakon, ausgewählt aus ¹⁵O-H₂O-Liganden, ¹¹C-McN 5652, (S)-([18F]fluoromethyl)-(+)-McN5652, ¹¹C-DASB und ¹¹C-DAPP sein. Alternativ kann ein ebenfalls bevorzugtes Radiopharmakon ein herkömmliches SPECT-Radiopharmakon, ausgewählt aus ¹²³I--ADAM ¹²³I--IDAM, ¹²³I--ODAM, ¹²³Iß-CIT und ¹²³I-nor-ß-CIT sein. Natürlich können auch andere Radiopharmaka verwendet werden, die dem Fachmann im Stand der Technik und aus der Literatur bekannt sind.

Erfindungsgemäß wurde überraschend gefunden, dass bei Verwendung eines Radiopharmakon zur Messung der Verfügbarkeit des SERT in der pontomesencephalen Region des Gehims, die die Region des rostralen Raphe-Kemkomplexes umfasst, bei Migränepatienten eine deutliche Erhöhung der Verfügbarkeit des Serotonin-Transport-Proteins ("SERT") in der pontomesencephalen Region, der anatomischen Region des rostralen Raphe-Kernkomplexes, über eine erhöhte Radioaktivitätsspeicherung nachzuweisen war. Auch wenn nur ein Radiopharmakon, welches spezifisch an den SERT bindet (¹²³I-ADAM), für diese Untersuchung eingesetzt wurde, ist aus wissenschaftlicher Sicht davon auszugehen, dass sich die Ergebnisse mit jedem Radiopharmakon, welches spezifisch am Serotonintransporter bindet, reproduzieren lassen. Dies bedeutet, dass erfindungsgemäß bevorzugt das entsprechende Radiopharmakon in der Therapieverlaufskontrolle der Migräne, des Clusterkopfschmerzes oder des arzneimittelinduzierten Kopfschmerzes verwendet werden kann.

In einem weiteren Aspekt davon betrifft die vorliegende Erfindung dann ein in vitro Verfahren zum Screening auf Substanzen, die die Bindung eines spezifisch an das Serotonin-Transport-Protein (SERT) bindenden Radiopharmakons modulieren, umfassend die Schritte von: a) zur Verfügung stellen eines in vitro-Systems, welches den SERT und das SERT-spezifische Radiopharmakon umfaßt, b) zur Verfügung stellen einer Kandidaten-Substanz, c) in Kontakt bringen von SERT und SERT-spezifischem Radiopharmakon zusammen mit der Kandidaten-Substanz und ohne die Kandidaten-Substanz, und d) Messen der Bindung zwischen SERT und SERT-spezifischem Radiopharmakon, wobei ein Unterschied in der Bindung mit der Kandidaten-Substanz und ohne die Kandidaten-Substanz eine Substanz identifiziert, die die Bindung eines spezifisch an das Serotonin-Transport-Protein (SERT) bindenden Radiopharmakons modifiziert. Erfindungsgemäß kann die Modulation eine Inhibierung oder Erhöhung der Bindung des Radiopharmakons an den SERT sein.

Von entsprechenden, die Bindung eines spezifisch an das Serotonin-Transport-Protein (SERT) bindenden Radiopharmakons modifizierenden Verbindungen wird erwartet, dass sich diese als Therapeutika oder zumindest als Vorstufen ("lead compounds") für die Behandlung von Kopfschmerzen, insbesondere Migräne, Clusterkopfschmerz und/oder arzneimittelinduzierten Kopfschmerz eignen. Besonders bevorzugt sind dabei identifizierte Substanzen, die eine selektive Modulation von SERT des in vitro-Systems aus der pontomesencephalen Region des Gehirns und insbesondere aus den serotonergen Raphe-Kernen bewirken, da diese als auf Basis der Ergebnisse im Rahmen der vorliegenden Erfindung als besonders für die Behandlung von Kopfschmerzen, insbesondere Migräne, geeignet scheinen.

Ein "in vitro-System" im Sinne der vorliegenden Erfindung ist ein für das oben genannte Screening-Verfahren geeignete Anordnung oder Vorrichtung, welche die Messung der Bindung eines spezifisch an das Serotonin-Transport-Protein (SERT) bindenden Radiopharmakons ermöglicht. Das System umfaßt zumindest das SERT und das SERT-spezifische Radiopharmakon, gegebenenfalls liegt das SERT in einem, z.B. rekombinanten, Zellmodell vor, mittels dessen dann die Modulation ermittelt werden kann. Gemessen werden kann z.B. zum einen die Menge des gebundenen (SERT) bindenden Radiopharmakons selber, das bevorzugt ausgewählt ist aus herkömmlichen PET- oder SPECT-Radiopharmaka (siehe oben). Weiterhin kann ein indirekter Nachweis über das SERT erfolgen, z.B. über Uptake-Bestimmungen von Serotonin (oder Derivaten davon) oder über markierte Antikörper, die an das SERT oder das Radiopharmakon binden. Auch kann die Kandidaten-Substanz entsprechend markiert sein. Alle Bestandteile des in vitro-Systems der vorliegenden Erfindung können in Form eines Kits vorliegen, mittels dem dann das erfindungsgemäße Screening durchgeführt werden kann.

Als Kandidaten-Substanzen können alle im Rahmen eines Screenings geeigneten Substanzen verwendet werden, insbesondere Substanzen niederen Molekulargewichts ("small molecules"), die z.B. in entsprechend käuflich zu erhaltenden Substanzbibliotheken erhältlich sind. Mögliche Substanzen sind auch Betablocker Metoprolol, Propranolol, Flunarizin oder Valproinsäure, deren entsprechende Verwendung zur spezifischen Kopfschmerz- oder Migränetherapie festgestellt werden kann.

Ein weiterer Aspekt der vorliegenden Erfindung betrifft dann ein Verfahren zur Herstellung eines Pharmazeutikums zur Behandlung von Kopfschmerzen, umfassend ein Screening-Verfahren wie oben definiert, und Mischen der identifizierten Substanz mit einem geeigneten pharmazeutischen Träger. Ein weiterer Aspekt der vorliegenden Erfindung betrifft schließlich eine pharmazeutische Zusammensetzung (Pharmazeutikum), umfassend eine erfindungsgemäß identifizierte Substanz, zusammen mit geeigneten Zusatz- oder Hilfsstoffen. Diese pharmazeutische Zusammensetzung kann dadurch gekennzeichnet sein, daß die Substanz in Form einer Depotsubstanz zusammen mit einer geeigneten, pharmazeutisch verträglichen Verdünnungslösung oder Trägersubstanz vorliegt.

Bevorzugt ist außerdem eine pharmazeutische Zusammensetzung gemäß der vorliegenden Erfindung, die weitere Therapeutika enthält. Diese Therapeutika können alle für den Fachmann üblichen Therapeutika im Rahmen einer Schmerztherapie umfassen und z.B. Betablokker wie etwa Metoprolol, Propranolol, Flunarizin oder Valproinsäure sein.

Erfindungsgemäß kann die oben genannte pharmazeutische Zusammensetzung in Form von Tabletten, Dragees, Kapseln, Tropflösungen, Suppositorien, Injektions- oder Infusionszubereitungen zur peroralen, rektalen oder parenteralen Verwendung vorliegen. Solche Darreichungsformen und deren Herstellung sind dem Fachmann bekannt.

Ein weiterer Aspekt der vorliegenden Erfindung betrifft die Verwendung mindestens einer der oben genannten identifizierte Substanzen zur Behandlung von Erkrankungen, wie z.B. Kopfschmerzen, insbesondere Migräne, des Clusterkopfschmerzes und/oder des arzneimittelinduzierten Kopfschmerzes. Diese Verwendung kann zum Beispiel in Form einer Depotsubstanz zusammen mit einer geeigneten, pharmazeutisch verträglichen Verdünnungslösung oder Trägersubstanz erfolgen. Ein weiterer Aspekt ist die Verwendung einer oder mehrerer der erfindungsgemäßen Verbindung(en) Herstellung eines Medikaments zur Behandlung von Erkrankungen wie z.B. Kopfschmerzen, insbesondere Migräne, des Clusterkopfschmerzes und/oder des arzneimittelinduzierten Kopfschmerzes. Diese Herstellung kann analog zu der oben beschriebenen Weise erfolgen.

Die vorliegende Erfindung betrifft weiterhin ein Verfahren zur Behandlung einer Erkrankung, ausgewählt aus Kopfschmerzen, insbesondere Migräne, des Clusterkopfschmerzes und/oder des arzneimittelinduzierten Kopfschmerzes, die das Verabreichen einer erfindungsgemäß identifizierten Substanz, etwa in Form einer erfindungsgemäßen pharmazeutischen Zusammensetzung, umfaßt. Die Verabreichung kann erfindungsgemäß z.B. in Form einer Depotsubstanz zusammen mit einer geeigneten, pharmazeutisch verträglichen Verdünnungslösung oder Trägersubstanz erfolgen.

Das von den Erfindern beschriebene Untersuchungsresultat eröffnet weiterhin die Möglichkeit, SERT-Radiopharmaka nicht nur in der Diagnose und Differentialdiagnose von Kopfschmerzerkrankungen, sondern auch im Therapiemonitoring einzusetzen. Wie oben bereits beschrieben, werden in der Migräneprophylaxe serotonerge Substanzen wie beispielsweise das Amitriptylin eingesetzt. Neben Amitriptylin stehen jedoch noch eine Reihe weiterer Migräneprophylaktika wie beispielsweise die Betablocker Metoprolol und Propranolol, der Calciumantagonist Flunarizin oder das Antikonvulsivum Valproinsäure zur Verfügung. Die Wirkweise dieser Prophylaktika ist bisher völlig unklar. Die prophylaktische Therapie der Migräne wird generell durch die Tatsache erschwert, dass das "Ansprechen" des Patienten auf ein vom Arzt gewähltes Prophylaktikum nicht vorhersehbar ist. Dies führt dazu, dass häufig mehrere Medikamente "ausprobiert" werden müssen, bevor eine geeignete, wirksame Substanze gefunden wird. Die Verwendung von SERT-Radiopharmaka könnte dabei helfen, das Wirkprinzip der Migräneprophylaktika in Hinblick auf ihren Einfluss auf das serotonerge System zu untersuchen und die Ursache für das "Non-Responding" von Patienten auf Prophylaktika zu klären. Darüber hinaus erscheinen SERT-Radiopharmaka auch als geeignet für ein Therapiemonitoring.

Eine weitere Anwendungsmöglichkeit für den Einsatz von SERT-Radiopharmaka ist die Diagnose und Differentialdiagnose des arzneimittelinduzierten Kopfschmerzes.. Nach epidemiologischen Daten ist die Migräne die häufigste primäre Kopfschmerzerkrankung, die einem arzneimittelinduzierten Kopfschmerz zugrunde liegt. An zweiter Stelle liegt der chronische Spannungskopfschmerz (Evers et al., 1999). Der arzneimittelinduzierte Kopfschmerz hat eine erhebliche ökonomische Relevanz. Zum einen führt er aufgrund des hohen Leidensdrucks der Patienten zu häufigen Ausfällen am Arbeitsplatz, zum anderen entstehen hohe Therapiekosten, da bei den Patienten entweder eine 5 bis 10-tägige stationäre Entzugsbehandlung durchgeführt wird, oder der Patient für die Dauer eines ambulanten Entzuges krankgeschrieben wird. Wird der arzneimittelinduzierte Kopfschmerz nicht rechtzeitig erkannt, kann dies zu irreversiblen Nierenschäden mit der Konsequenz einer lebenslangen Dialysepflichtigkeit oder der Nierentransplantation führen. Die Diagnose des arzneimittelinduzierten Kopfschmerzes ist oft sehr schwierig, da sie lediglich auf den - nicht immer verlässlichen Angaben des Patienten - über die monatliche Schmerzmitteleinnahme beruht. Abgesehen von der Medikamentenanamnese gibt es derzeit keine weiteren Kriterien für die differentialdiagnostische Unterscheidung zwischen einem chronischen Spannungskopfschmerz oder einer chronischen Migräne ohne Schmerzmittelabusus und einem arzneimittelinduzierten Kopfschmerz. Wie oben ausgeführt gibt es indirekte Hinweise für eine serotonerge Dysregulation bei Patienten mit arzneimittelinduziertem Kopfschmerz. SERT-Radiopharmaka könnten daher auch hier diagnostisch und differentialdiagnostisch zum Einsatz kommen.

### 2. Einsatz der Erfindung

Das von den Erfindern beschriebene Untersuchungsverfahren eignet sich somit für den Einsatz in der klinischen Routine. Zusammenfassend eröffnet die vorliegende Erfindung folgende Möglichkeiten der Anwendung:

### 2.1. Diagnose der Migräne und differentialdiagnostische Abgrenzung der Migräne

Die Ergebnisse von mit dem Radiopharmakon ¹²³I-ADAM durchgeführten Untersuchungen haben bei Migränepatienten eindeutig eine signifikante Erhöhung der Verfügbarkeit dieses Radiopharmakons in der pontomesencephalen Region gezeigt. Daher werden sich SERT spezifische Radiopharmaka für die bildgebend-funktionelle Diagnose der Migräne eignen. Die häufigste Fehldiagnose der Migräne ist der Spannungskopfschmerz. Die Pathophysiologie des Spannungskopfschmerzes ist bisher weitestgehend unbekannt. Die medikamentösen Therapieansätze zur Behandlung von Migräne und Spannungskopfschmerz weichen erheblich voneinander ab. Zwar ist Amitriptylin nicht nur bei der Präventionsbehandlung der Migräne, sondern auch in der Prophylaxe des Spannungskopfschmerzes wirksam, im Gegensatz zur Migräne sind ***Serotonin-Agonisten*** in der Akuttherapie des Spannungskopfschmerzes jedoch ***unwirksam.*** Dies deutet darauf hin, dass das serotonerge System beim Spannungskopfschmerz lediglich eine **sekundäre unspezifische,** nicht jedoch eine ursächliche pathophysiologische Rolle spielt. Aus unserer Sicht ist daher damit zu rechnen, dass sich Migräne und Spannungskopfschmerz in Hinblick auf die Verfügbarkeit des Serotonin-Transportproteins im Gehirn vollständig unterscheiden. Somit könnte im Rahmen von Untersuchungen mit SERTspezifischen Radiopharmaka eine Differentialdiagnostik der beiden Erkrankungen gelingen.

### 2.2. Differentialdiagnose des Clusterkopfschmerzes

Nach bisherigen Erkenntnissen zur Wirksamkeit von serotonergen Substanzen in der Clusterkopfschmerztherapie spielt das Serotonin eine wesentliche Rolle in der Pathophysiologie des Clusterkopfschmerzes. Nuklearmedizinische Untersuchungen haben gezeigt, dass sich bei Clusterkopfschmerzpatienten in einem typischen Projektionsgebiet serotonerger Neurone, dem Hypothalamus, eine Erhöhung des Blutflusses zeigt. Somit erscheint es möglich, mit dem Einsatz von Radiopharmaka, die spezifisch an den SERT binden, eine für die Diagnose des Clusterkopfschmerzes spezifische serotonerge Dysregulation im Hypothalamus nachzuweisen.

### 2.3. Pathophysiologie des arzneimittelinduzierten Kopfschmerzes

Die häufigste sekundäre Kopfschmerzerkrankung ist der arzneimittelinduzierte Kopfschmerz. Dieser führt auch heute noch in vielen Fällen aufgrund medikamentös-toxischer Nierenschäden bei den Patienten zur Dialysepflichtigkeit oder zur Nierentransplantation. Damit stellt der arzneimittelinduzierte Kopfschmerz auch volkswirtschaftlich ein erhebliches Problem für das Gesundheitswesen dar. Die Diagnose des arzneimittelinduzierten Kopfschmerzes beruht derzeit lediglich auf klinischen Angaben. In Thrombozytenuntersuchungen von Patienten mit arzneimittelinduziertem Kopfschmerz zeigten sich Hinweise auf einen massiven Serotoninmangel bei den Betroffenen. Das von den Erfindern beschriebene Untersuchungsverfahren soll daher zunächst eingesetzt werden, um die Hypothese einer Dysregulation des Serotonin-Transportproteins bei Patienten mit Analgetika induziertem Kopfschmerz zu überprüfen. Später könnte die Methode auch zur Diagnostik und Verlaufskontrolle von Patienten mit Schmerzmittelmissbrauch eingesetzt werden. Darüber hinaus erscheint der Einsatz von Radiopharmaka, die spezifisch am SERT binden, für eine differentialdiagnostische Unterscheidung zwischen einer primären Kopfschmerzerkrankung (chronischer Spannungskopfschmerz, chronische Migräne) und dem sekundären arzneimittelinduzierten Kopfschmerz sinnvoll.

### 2.4 Verlaufsuntersuchungen mittels spezifisch an den SERT bindenden Radiopharmaka und - SPECT unter medikamentöser Migränetherapie

Migränepatienten profitieren sowohl in der Akuttherapie als auch der Prophylaxe von serotonergen Substanzen. Das von den Erfindern beschriebene Untersuchungsverfahren könnte in Zukunft Relevanz im Drug-Monitoring und bei der Identifizierung von Patienten, die auf die Standard-Medikation nicht ansprechen, haben.

Die Erfindung soll nun im folgenden unter Bezug auf die beigefügten Abbildungen genauer beschrieben werden, ohne jedoch auf die speziellen Beispiele beschränkt zu werden.

### 3. Beispiele

Folgende Hypothese wurde im Rahmen der Untersuchung geprüft: "Migränepatienten zeigen im Vergleich zu gesunden, alters- und geschlechts-angepassten Kontrollpersonen eine signifikante Veränderung der Serotonin-Transporter-Bindung (SERT-Bindung) in dem mit hoher Dichte serotonerg innervierten pontomesencephalen Hirnstamm (Region des rostralen Raphe-Kemkomplexes)". Die Serotonin-Transporterbindung wurde dabei mit Hilfe der ¹²³I-ADAM-SPECT semiquantitativ bestimmt.

Zielparameter war die semiquantitative Ermittlung der spezifischen ¹²³I-ADAM-Aufuahme in ROI-Technik im Bereich des pontomesencephalen Hirnstamms (Raphe-Kerne) durch die ¹²³I-ADAM-Himszintigraphie in SPECT-Technik 4 Stunden nach intravenöser Injektion.

### 3.1 Verwendetes Radiopharmakon

Für die nuklearmedizinisch-fünktionelle Bildgebung des serotonergen Systems im zentralen Nervensystem wurde das Radiopharmakon ^{I23}I-ADAM eingesetzt (Oya et al. 2000). Das Radiopharmakon ¹²³I-ADAM ist kommerziell erhältlich, arzneimittelrechtlich derzeit jedoch noch nicht zugelassen. Das Radiopharmakon wurde bisher umfangreich pharmakologischtoxikologisch getestet. Es konnte gezeigt werden, dass der Einsatz dieser Substanz am Menschen sicher ist (Oya et al. 2000, Choi et al. 2000; Acton et al. 2001, Ahonen et al. 2002 a und b, Kauppinen et al. 2003). Vor jeder Anwendungdes Radiopharmakons ¹²³I-ADAM am Menschen werden vom Hersteller (MAP Medical Technologies Oy, Elementtitie 27, 41160 Tikkakoski, Finnland) Prüfungen auf radiochemische- und Radionuklidreinheit, auf pH-Wert und auf Sterilität vorgenommen. Insgesamt wurden bisher keine Nebenwirkungen oder sonstige unerwünschte Ereignisse bei der Anwendung am Menschen mit dem Radiopharmakon ¹²³I-ADAM beobachtet.

### 3.2 Patienten/Probanden

Es wurden 19 Patienten mit Migräne, diagnostiziert anhand der Diagnosekriterien der Internationalen Kopfschmerzgesellschaft, untersucht (17 Patienten mit Migräne ohne Aura, 2 Patienten mit Migräne mit Aura). Als Kontrollgruppe wurden 9 gesunde Probanden untersucht. Patienten und Kontrollpersonen wurden alters- und geschlechts-angepasst ausgewählt (Migräne: w: m = 17 : 3, Alter: 43,4 ±117, Kontrollen: w: m = 6 : 3; Alter: 42,6 ± 10,2).

Da sich in nuklearmedizinischen Untersuchungen bisher unsystematische Hinweise auf eine Veränderung der SERT-Verfügbarkeit bei Patienten mit einer majoren Depression ergaben (Malison et al., 1998; Pirker et al.,1995,Willeit et al., 2000), wurde bei allen Patienten und Kontrollpersonen eine psychiatrische Anamnese erhoben, um psychiatrische Erkrankungen als möglichen Einflussfaktor unserer Untersuchungen zu vermeiden. Darüber hinaus wurden standardisierte Fragebögen zum Ausschluss einer Angststörung und einer Depression vorgelegt. Weder bei den Patienten noch bei den Kontrollpersonen ergaben sich Hinweise auf eine psychiatrische Erkrankung.

### 3.2.1 Einschlusskriterien

### Patienten mit Migräne-Kopfschmerz

- Patienten (m o. w) im Alter zwischen 18-60 Jahren
- dokumentierte klinische Diagnose einer Migräne ohne Aura bzw. Migräne mit Aura bzw. eines Clusterkopfschmerzes vom episodischen Typ
- Vorliegen der Diagnosekriterien nach ICD-10:
   - Migräne mit Aura: G 43.1
   - Migräne ohne Aura: G. 43.0
- keine prophylaktische Behandlung des Kopfschmerzsyndroms innerhalb der letzten 8 Wochen vor Einschluss in die Studie
- die Patientin/der Patient muss in der Lage sein, die Patienteninformation zu verstehen, die erforderlichen Untersuchungen bzw. Termine einzuhalten und eine schriftliche Einverständniserklärung geben zu können (Einwilligungsfähigkeit).

### Gesunde Probanden:

- freiwillige Probanden (m o. w) im Alter zwischen 18 und 60 Jahren;
- altersentsprechender guter Gesundheitszustand, dokumentiert im Rahmen der klinischen Untersuchung bei Screening.
- in der Eigenanamnese keine neurologischen oder psychiatrischen Krankheiten in der Vorgeschichte.
- Der Proband/die Probandin muss in der Lage sein, die Studieninformation zu verstehen, die erforderlichen Untersuchungen und Termine einzuhalten und eine schriftliche Einverständniserklärung geben zu können.

### 3.2.2 Ausschlusskriterien für Patienten

### Allgemeine Ausschlusskriterien:

- Einnahme von Medikamenten, die mit der Aufnahme des Radiopharmakons ¹²³I-ADAM interferieren könnten (unerlaubte Medikamente)
- Beeinträchtigung der Nieren- oder Leberfunktion, die die Pharmakokinetik des ¹²³I-ADAM beeinflussen könnte: Nierenerkrankungen mit um mehr als das 3fache der Norm erhöhten Serum-Kreatmin-Spiegeln, Lebererkrankungen mit Transaminasen-Erhöhung um mehr als das 3-fache der Norm und/oder γ-GT-Erhöhung um mehr als das 5 fache der Norm
- berufliche oder sonstige Strahlenexposition >15mSv pro Jahr
- Drogen- oder Alkoholmissbrauch, zum Zeitpunkt der Studie oder in der Vorgeschichte
- Teilnahme an einer anderen nuklearmedizinischen Untersuchung innerhalb von 5 Halbwertszeiten der eingesetzten Substanz vor Screening
- Frauen in Schwangerschaft oder Stillzeit

### Spezielle Ausschlusskriterien:

- Parkinsonsyndrome (M. Parkinson, Multisystematrophie, progressive supranukleäre Blickparese)
- zerebrovaskuläre Erkrankungen, ZNS-Tumore, Normaldruckhydrozephalus
- Demenz (DSM IV/ICD 10-Kriterien)
- wiederholte zerebrale Ischämien mit einer stufenweisen Verschlechterung
- Enzephalitis in der Vorgeschichte
- Hinweise auf strukturelle Schädigung der Basalganglien oder des Hirnstamms
- Vorliegen einer depressiven Erkrankung (ICD 10 F 32, F33)
- Vorliegen einer generalisierten Angststörung (ICD 10 F 41.1)
- Komorbidität mit anderen primären Kopfschmerzerkrankungen: Kopfschmerz vom Spannungstyp (ICD 10 G. 44.2), Clusterkopfschmerz (ICD 10 G 44.0) bei Migränepatienten, - Komorbidität mit Gesichtsschmerzen (z. B. atypischer Gesichtsschmerz, ICD 10 G 50.1), Trigeminusneuralgie (ICD 10 G. 50)

### 3.2.3 Abbruchkriterien

Die Studie konnte jederzeit von dem behandelndem Arzt aus wichtigem Grund beendet werden. Wichtige Gründe sind z.B. unvorhergesehene, nicht tolerierbare Nebenwirkungen. Patienten wurden aus der Studie genommen, wenn
- ein schwerwiegendes oder medizinisch intolerables unerwünschtes Ereignis auftrat,
- Symptome einer Erkrankung auftraten, die unter die Ausschlußkriterien fällt,
- dies der Wunsch des Patienten war,
- die Compliance des Patienten nicht ausreichte.

### 3.2.4 Unerlaubte Medikamente (bekannte Interaktion mit ¹²³I- ADAM):

- ZNS-Stimulantien: Kokain, Amphetamine, Methylamphetamin, Methylphenidat
- Medikamente zur Therapie der Anorexie / Obesitas: Phentermine, Mazindol, Dextroamphetamin
- Sympathomimetika: Norephedrine, Phenylpropanolamine
- Antidepressiva, speziell Substanzklassen der SSRI, SNRI, NASSA, NARI
- Anti-Parkinsonmedikamente (L-Dopa, Dopaminagonisten, MAO-/COMT-Hemmer, Amantadine, Budipine)
- 5HT 1B/1D-Rezeptoragonisten ("Triptane") bis zu 48 Stunden vor Beginn der Untersuchung

### 3.3 Projektbedingte Untersuchungen

Screening: Die Aufnahme der Patienten/Probanden in die Studie erfolgte im Rahmen eines Screenings in der Klinik für Neurologie, bei dem nach Anamnese und neurologischer/psychiatrischer Untersuchung die Überprüfung der Diagnose sowie der Ein- und Ausschlußkriterien erfolgte. Die Patienten wurden über die geplanten Untersuchungen mündlich und schriftlich aufgeklärt. Vor Einschluss in die Studie war das schriftliche Einverständnis erforderlich.

### Visitenplan für Probanden und Migränepatienten:

Visite 1: Frühestens am Folgetag nach Screening (1 Tag bis 2 Wochen) erhielten die Patienten in der Klinik für Nuklearmedizin die intravenöse Injektion des Radiopharmakons ¹²³I-ADAM. Im Anschluß an die Injektion wurden die Patienten nachbeobachtet (Kreislauf, Blutung an der Injektionsstelle).

¹²³I-ADAM-SPECT: 4 Stunden nach Injektion des Radiopharmakons wurde die ¹²³I-ADAM-SPECT-Untersuchung in der Klinik für Nuklearmedizin durchgeführt. Eine Untersuchung dauerte ca. 45 min. Die Patienten/Probanden wurden nach eventuellen Nebenwirkungen/unerwünschten Wirkungen nach der Injektion befragt.

Visite 2: Am Tag nach der nuklearmedizinischen Untersuchung erfolgte ein follow-up, d.h. die Patienten/Probanden wurden nochmals hinsichtlich unerwünschter Wirkungen/Ereignissen evaluiert. Diese Befragung ist bei ambulanten Patienten telefonisch erfolgt.

### 3.4 Untersuchungsvorgang.

Für die SPECT-Untersuchung erhielt der Patient (nach vorheriger Blockade der Schilddrüse mit Perchlorat) eine intravenöse Injektion von 130 MBq ¹²³I-ADAM. 4 Stunden nach Injektion wurde ein ADAM-Hirn-SPECT durchgeführt. Die ¹²³I-ADAM-Hirn-SPECT-Untersuchung wurde an einer Doppelkopflcamera (MultiSpect2, Fa. Siemens) mit einem Low Energy High Resolution Kollimator (LEHR) mit einer Matrix von 128 x 128 mit 120 Projektionen (60/Kamerakopf) bei einem Winkelschritt von 3 Grad und einer Zeit von 40 Sekunden/Projektion ohne Zoom durchgeführt. Anschließend wurden die SPECT-Rohdaten iterativ mit der kommerziell verfügbaren Rekonstruktionssoftware ReSPECT (Firma SCIVIS) rekonstruiert.

Für die anatomische Zuordnung der SPECT-Ergebnisse wurde von den Patienten am gleichen Untersuchungstag eine zerebrale Kernspintomographie durchgeführt (1,5 Tesla, Fa. Siemens). Anschließend wurde eine Fusion der dreidimensionalen kernspintomographischen Bilder und der iterativ rekonstruierten ¹²³I-ADAM-Hirn-SPECT-Daten mit Hilfe der kommerziell verfügbaren Software MPI-Tool der Firma Advanced Tomo Vision durchgeführt.

### 3.5 Zielregion:

Als Zielregion wurden die Ursprungskerne serotonerger Neurone, die sogenannten "Raphe-Kerne" gewählt. Der rostrale Raphe-Kernkomplex liegt im Mittelhirn und in der Brücke ("pontomesencephale" Region) (Törk, 1990). (Der kaudale Raphe-Kemkomplex ist für Untersuchungen des Serotoninstoffwechsels im Gehirn nicht von Bedeutung, da dieser Kemkomplex ins Rückenmark und nicht ins Gehirn projiziert). Für die statistische Auswertung wurde daher jeweils die gesamte anatomische Region von Mittelhirn und Pons gewählt.

### 3.6 Auswertung der Daten:

Die Zielregion ist, wie oben beschrieben, der pontomesencephale Hirnstamm. Um den Gesamtuptake von ¹²³I-ADAM in der Zielregion zu ermitteln, wurde dabei zunächst schichtweise in den transversalen Aufnahmen der fusionierten SPECT/MRT-Schnitte die "Region of Interest" (ROI) eingezeichnet. Der Gesamtuptake der Zielregion wurde nach folgender Vorgehensweise bestimmt: Zunächst wurden alle counts/pixel mit den Gesamtpixel einer ROI multipliziert (Gesamtimpulse x Gesamtpixel). Die Gesamtimpulse einer ROI wurden danach über alle Schichten aufsummiert (Gesamtimpulse der Zielregion). Parallel dazu wurde das Gesamtvolumen der Zielregion bestimmt. Die Gesamtimpulse der Zielregion wurden abschließend durch das Gesamtvolumen der Zielregion dividiert. Der so erhaltene Aktivitätsquotient (AQ in counts/mm³) ist der AQ, der in die Gesamtberechnung eingeht.

Wahl der Referenzregion: In den bisherigen nuklearmedizinischen Untersuchungen zur Bestimmung der SERT-Dichte wurde das Kleinhirn als Referenzregion gewählt (Oya et al., 2000; Shioe et al., 2003; Buchert et al., 2003). In der visuellen Auswertung der erfindungsgemäßen ¹²³I-ADAM-Him-SPECT-Untersuchung bei Migränepatienten zeigte sich jedoch im Kleinhirn eine relevante Anreicherung des Radiopharmakons, die über der Untergrundspeicherung lag. Da das Kleinhirn damit als Referenzregion nicht geeignet erschien, wurde als Alternative eine anatomische Region gewählt, deren Uptake im Bereich der üblichen Untergrundspeicherung lag. Als geeignet für die Referenzregion zeigte sich dabei der Okzipitallappen. Analog zur Zielregion (siehe oben) wurde ein Aktivitätsquotient für die Referenzregion errechnet. Der endgültige Zielparameter als Äquivalent der SERT-Dichte wurde aus den Aktivitätsquotienten nach folgender Formel ermittelt:

### 3.7 Zielregion (Pontomesencephale Region) - Referenzregion (Okzipitallappen)/Referenzregion

Die in den ¹²³I-ADAM-Untersuchungen erhaltenen Aktivitätsquotienten wurden einer statistischen Analyse (zweiseitiger t-Test für unverbundende Stichproben) unterzogen.

### 3.8 Ergebnisse:

Bei den gesunden Kontrollpersonen wurde ein Aktivitätsquotient (Zielregion - Referenzregion/Referenzregion) von 0,57 +/- 0,18 ermittelt. Im Gegensatz dazu war bei den Migränepatienten der Aktivitätsquotient auf 0,88 +/- 0,16 erhöht. Dieser Unterschied war im Rahmen eines zweiseitigen t-Tests für unverbundene Stichproben hochsignifikant (p < 0,01). Die Ergebnisse der Untersuchungen sind in Abbildung 1 bis 5 wie folgt dargestellt:

Abbildung 1: Darstellung der Aktivitätsquotienten von gesunden Kontrollen und Migränepatienten in einer Box-Plot-Analyse. Der Unterschied zwischen den beiden Untersuchungsgruppen war hoch signifikant (p < 0,01). Im vorliegenden Fall wurde im Rahmen der vorliegenden Erfindung eine Erhöhung des Verhältnisses des SERT bei Migränepatienten von pontomesencephal zu occipital zwischen etwa 0,8 bis 1,0 und insbesondere 0,85 festgestellt. Entsprechende Veränderungen der Verhältnisse werden auch in anderen Kopfschmerzformen und mit anderen verwendeten Radiopharmaka und Untersuchungsverfahren erwartet.

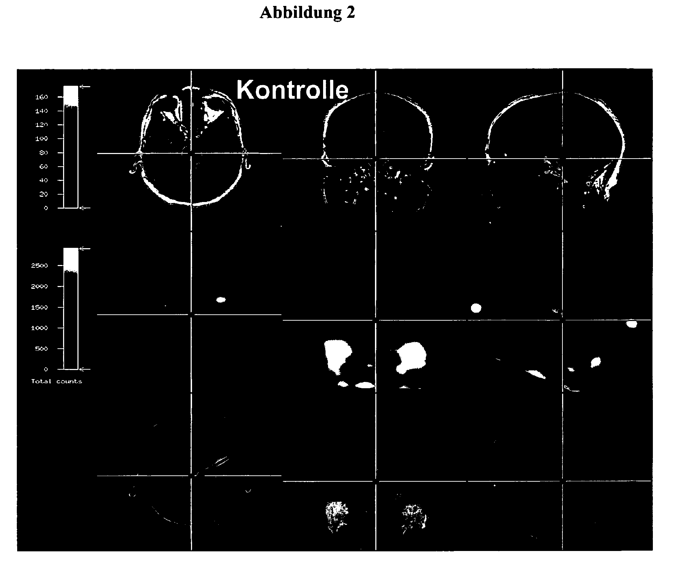

Die Abbildungen 2 bis 5 zeigen Beispiele für Ergebnisse von gesunden Probanden (Kontrollen) und Patienten aus verschiedenen Bereichen des Hirns. In der obersten Reihe ist jeweils die zerebrale Kernspintomographie der untersuchten Personen zu sehen, die mittlere Reihe stellt jeweils die Ergebnisse der SPECT-Untersuchung dar. Für eine Lokalisationsanalyse der SPECT-Daten wurde eine Bildfusion aus Kernspintomographie und SPECT durchgeführt. Die unterste Reihe stellt jeweils diese Fusionsbilder dar. Die ausgewählten Hirnschnitte sind dreidimensional dargestellt: In der linken Spalte finden sich die Transversalaufnahmen, in der mittleren Spalte sind die koronaren Schichten und in der rechten Spalte sind die Sagittalaufnahmen dargestellt. Die Pfeile markieren die Zielregion (pontomesencephale Region). Bereits in der visuellen Analyse wird eine erhöhte Verfügbarkeit von SERT durch eine erhöhte Aktivitätsspeicherung in der Zielregion von Patienten, verglichen mit Kontrollen, sichtbar.

Abbildung 2: In der unteren Reihe von Abbildung 2 sind die Fusionsbilder (SPECT und MRT) einer gesunden Kontrollperson dargestellt. Der Pfeil im linken und rechten unteren Bild markiert die "Pons". In den Fusions-Aufnahmen der unteren Bildreihe kommt bei der Kontrollperson eine vergleichsweise geringe Aktivitätsspeicherung zur Darstellung.

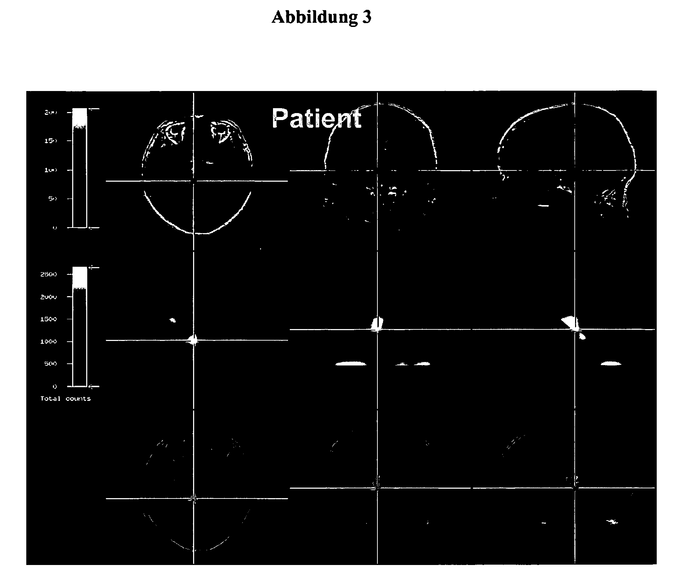

Abbildung 3 stellt die Fusionsbilder einer Patientin mit Migräne ohne Aura dar. In der unteren Bildreihe markieren die Pfeile wiederum die pontomesencephale Region. Im Vergleich zur Kontrollperson zeigt sich bereits visuell (vor allem in der Sagittalaufnahme rechts unten) eine deutlich erhöhte Aktivitätsspeicherung.

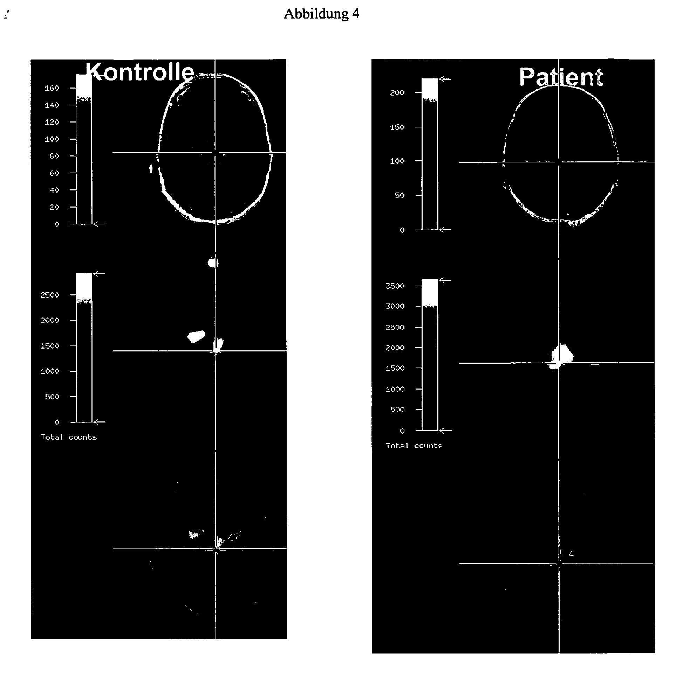

Abbildung 4: Um den Unterschied zwischen Kontrollpersonen und Migränepatienten zu verdeutlichen, sind die Ergebnisse einer Kontrollperson und einer Migränepatientin direkt gegenübergestellt. Die hier dargestellten Transversalaufnahmen zeigen die Region des Mesencephalon (roter Pfeil). Der Unterschied in der Aktivitätsspeicherung zwischen Kontrollperson und Patientin ist deutlich zu sehen.

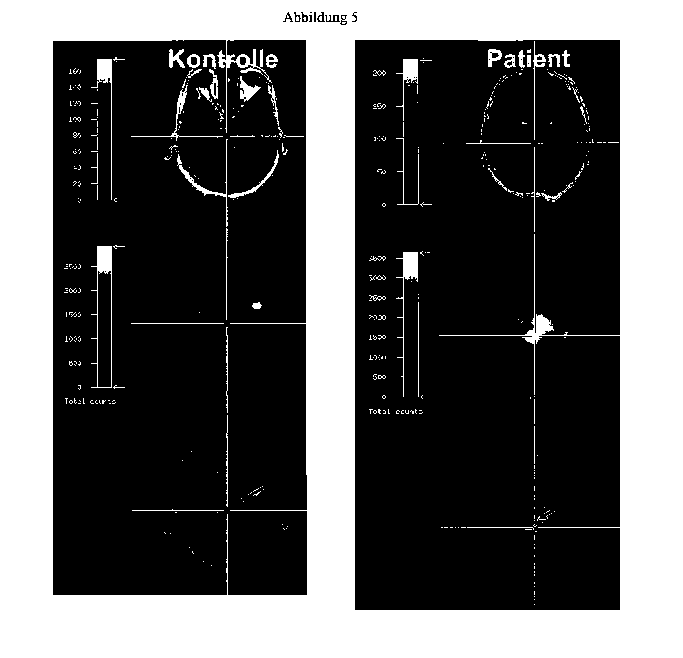

In Abbildung 5 ist in den Transversalaufnahmen die Region der "Pons" zu sehen (roter Pfeil). Auch hier wird ein deutlicher Unterschied in der markierten Region zwischen gesunder Kontrollperson und Migränepatient sichtbar.

### 4. LITERATUR:

* Acton PD, Choi SR, Hou C, Plossl K, Kung HF Quantification of serotonin transporter; in nonhuman primates using [123I]-ADAM and SPECT. J Nucl Med. 2001;42:1556-62,
* Acton PD, Kung MP, Mu M, Plossl K, Hou C, Siciliano M, Oya S, Kung HF. Single-photon emission tomography imaging of serotonin transporter; in the non-human primate brain with the selective radioligand [121I]-ADAM. Eur J Nucl Med 1999,26:854-61.
* Ahonen AK, Kauppinen, TA, Heikman, P, Bergström KA, Launes, J, Nikkinen, P, Hiltunen, J. [123I]-labeled ADAM -a selective novel radioligand for imaging of serotonin transporters in the human brain. J Nucl Med 2002a; 43:232 P (Poster auf der 49. SNM-Tagung 2002).
* Ahonen AK, Kauppinen, TA, Heikman, P, Bergstrom, KA, Nikkinen, P, Kylkinen, T, Kamarainen, EL, Puronto, O, Wtuhan, J. Imaging of serotonin transporters in human beings using [123I]-ADAM. Eur J Nucl Med 2002b; 29 Suppl 1: 8120 (abstract 269, Poster EANM-Tagung 2002).
* Andersen E, Dafny N. Dorsal raphe stimulation reduces responses of parafascicular neurons to noxious stimulation. Pain 1983; 15: 323-331.
* Bahra A, May A, Goadsby PJ. Cluster headache: a prospective clinical study with diagnostic implications. Neurology 2002; 58: 354-361.
* Baumgarten, H.G., Göthert, M (eds.). Serotoninergic Neurons and 5-HT Receptors in the CNS. Springer Verlag Berlin, Heidelberg, 2000.
* Bergstrom KA, Halldin C, Hall H, Lundkvist C. Ginovart N, Swann CG, Farce L: In vitro and in vivo characterisation of nor-beta-CIT: a potential radioligand for visualisation of the serotonin transporter in the brain. Eur J Nuc1 Med. 1997;24:596-601.
* Bergstrom KA, Kuikka JT, Ahonen A, Vanninen E: [123I]-beta-CIT, a tracer for dopamine and serotonin re-uptake sites: preparation and preliminary SPECT studies in humans. J Nucl Biol Med 1994;38(St):128-31.
* Brücke T, Kornhuber J, Angelberger P, Asenbaum S, Frassine H, Podreka I. SPECT imaging of dopamine and serotonin transporters with [123I]-beta-CIT Binding kinetics in the human brain. J Neural Transm Gen Sect 1993; 94:137-46.
* Buchert R, Thomasius R, Nebeling B, Petersen K, Obrocki J, Jenicke L, Wilke F, Wartenberg L, Zapletalova P, Clausen M. Long-term effects of "Ecstasy" use on serotonin transporters of the brain investigated by PET: J Nucl Med 2003; 44: 375-384.
   Castillo J, Munoz P, Guitera V, Pascual J. Epidemiology of chronic daily headache in the general population. Headache 1999; 39: 190-196
* Choi SR, Hou C, Oya S, Mu M, Kung MP, Siciliano M, Acton PD, Kung HF. Selective in vitro and in vivo binding of [123I]-ADAM to serotonin transporters in rat brain. Synapse 2000; 38:403-12.
* Chugani DC, Niimura K Caturvedi S, Muzik O, Fakhouri M, Lee ML, Chugani HT. Increased brain serotonin synthesis in migraine. Neurology 1999; 53 (7):1473-9.
* D'Andrea, G., Cananzi, AR., Perini, F., Hasselmark, L. Platelet models and their possible usefulness in the study of migraine pathogenesis. Cephalalgia 1995; 15: 265-271 .
* Davies GM, Santanello N, Garth W, Lerner D, Block GA. Validation of a migraine work and productivity loss questionnaire for use in migraine studies. Cephalalgia 1999; 19: 497-502.
* Diener HC & Eikermann A. Behandlung der Migräneattacke. Aus: Kopfschmerzen (ed: Diener HC), Georg Thieme Verlag Stuttgart, New York 2003.
* Dodick DW. Rozen TD, Goadsby PJ, Silverstein SD. Cluster headache. Cephalalgia 2000, 20: 787-803.
* Dong WQ, Qiao JT, Skolnick M, Dafny N. Focal dorsal raphe stimulation and □ineal electrical stimulation modulation modulate spontaneous and noxious evoked responses in thalamic neurons. Int J Neurosci 1991; 57: 123-140.
* Ekbom K, Krabbe A, Micelli G, Prusinski A, Cole JA, Noronha D for the Stumatriptan Cluster Headache Long-term study group. Cluster headache attacks treated for up three months with subcutaneous sumatriptan (6mg). Cephalalgia 1995; 15: 230-236.
* Evers S, Suhr B, Bauer B, Grotemeyer KH, Husstedt W. A retrospective long-term analysis of the epidemiology and features of drug-induced headache. J Neurol. 1999; 246: 802-9.
* Ferrari MD & Saxena PR. On serotonin and migraine: a clinical and pharmacological review. Cephalalgia 1993; 13: 151-165.
* Ferrari MD, Roon KI, Lipton RB, Goadsby PJ. Oral triptans (serotonin 5HT 1B/1D agonists) in acute migraine treatment: a meta-analysis of 53 trials. Lancet 2001, 358: 1668-1675.
* Glass JD. Grossman GH, Farnbauch L, DiNardo L. Midbrain raphe modulation of nonphotic circadian clock resetting and 5-HT release in the mammalian suprachiasmatic nucleus. J Neurosci 2003; 23: 7451-7460.
* Hesse, S., Barthel, H., Schwarz, J., Sabri, O., Müller, U. Advances in in vivo imaging of serotonergic neurons in neuropsychiatric disorders. Neurosci. Biobehav. Rev. 2004; 28: 547-63.
* Hiltunen J, Akerman KK, Kuikka JT, Bergstrom KA, Halldin C, Nikula T, Rasanen P, Tiihonen J, Vauhkonen M, Karhu J, Kupila J, Lansimies E, Farde L: Iodine-123 labeled nor-beta-CIT as a potential tracer for serotonin transporter imaging in the human brain with single-photon emission tomography. Eur J Nucl Med 1998;25:19-23.
* Hill J, Akerman KK, Kuikka JT, Bergstrom KA, Halldin C, Nikola T, Rasanen P, Tiihonen J, Vauhkonen M, Karhu J, Kupila J, Lansimies E, Farce L: Iodine-123 labeled nor-beta-CIT as a potential tracer for serotonin transporter imaging in the human brain with single-photon emission tomography. Eur J Nucl Med 1998, 25:19-23.
* International Classification of Headache Disorders. Cephalalgia 2004; 24: Supplement 1 (2nd Edition).
* Jones DW, Gorey JIG, Zajicek K, Des S, Urbina R, Lee KS, Heinz A. Knable MB, Higley DR, Weinberger D, Linnoila M Depletion-restoration studies reveal the impact of endogenous dopamine and serotonin on [I-123]b-CIT SPECT imaging in primate brain. J Nucl Med 1998; 39 (Suppl):42.
* Jost W. Selbach O. Medikamentöse Akuttherapie. Aus: Therapie der Migräne (ed. Host W & Selbach O), Uni-Med Verlag Bremen- London- Boston. 2001.
* Juhasz, G. et al. Association analysis of 5-HTTLPR variants, 5-HAT (2A) receptor gene 102T/C polymorphism and migraine. J. Neurogenet. 2003; 17: 231-240
* Kauppinen, TA, Bergström, KA, Heikman, P, Hiltunen, J, Ahonen, AK. Biodistribution and radiation dosimetry of [123I]-ADAM in healthy human subjects: preliminary results. Eur J Nucl Med 2003; 30: 132-136.
* Kotani K, Shimomura T, Shimomura F, Ikawa S, Nanba E. A polymorphism in the serotonin transporter gene regulatory region and frequency of migraine attacks. Headache 2002, 42:893-895.
* Kung MP, Hou C, Oya S, Mu M, Acton PD, Kung HF. Characterization of [123I]-ADAM as a novel single-photon emission tomography tracer for serotonin transporters. Eur J Nucl Mad 1999; 26:844-53.
* Malison RT, Price LH, Berman R, VanDyck CH, Pelton GH, Carpenter L, Sanaccra G, Owens MJ, Nemeroff CB, Rajeevan N, Baldwin RM, Selby[ JP, Innis RB, Charney DS. Reduced brain serotonin transporter availability in major depression as measured by [123I]-2ß-carbomethoxy-3ß-(4-iodophenyl)tropane and Single Photon Emission Tomography. Biol Psychiatry 1998; 44: 1090-1098.
* May A, Barra A; Buchel C, Frackowiak RSJ, Goadsby PJ. Hypothalamus activation in cluster headache attacks. Lancet 1998;352: 275-278.
* Ogilvie AD, Russell MB, Dhall P, Battersby S, Ulrich V, Dale Smith CA, Goodwin GM, Harmar AJ, Olesen J. Cephalalgia 1998; 18: 23-26.
* Okada T, Fujita M, Shimada S, Sato K, Schloss P, Watanabe Y, Itoh Y, Tohyama M, Nishimura T: Assessment of affinities of beta-CIT, beta-CIT-FE, and beta-CIT-FP for monoamine transporters permanently expressed in cell lines. Nucl Med Biol 1998; 25:53-8.
* Oya S, Choi SR, Hou C, Mu M, Kung MP, Acton PD, Sicifano M, Kung HF. 2-((2-((dimethylamino)methyl)phenyl)thio)-5-iodophenylamine (ADAM): an improved serotonin transporter ligand. Nucl Med Biol 2000;27:249-54.
* Pirker W, Asenbaum S, Kasper S, Walter H, Angelberger P, Koch G, Pozzera A, Deecke L, Podreka I, Brocke T: beta-CIT SPELT demonstrates blockade of 5HT-uptake sites by citalopram in the human brain in vivo. J Neural Transm Gen Sect 1995;100:247-56.
* Prieto-Gomez B, Dafny N, Reyes-Vazquez C. Dorsal raphe stimulation, 5-HT and morphine microiontophoresis effects on noxious and nonnoxious identified neurons in the medial thalamus of the rat. Brain Res. 1989; 22: 937-943.
* Ramamoorthy, S. et al. Antidepressant- and cocaine-sensitive human serotonin transporter: molecular cloning, expression, and chromosomal localization. Proc Natl Acad Sci USA 1993; 90: 2542-2546
* Rasmussen BK. Migraine and tension-type headache in a general population: precipitating factors, female hormones, sleep pattern and relation to lifestyle. Pain 1993; 53. 65-72.
* Russell MB, Rasmussen BK, Thornvaldesen P, Grasso J. Prevalence and sex ratio of the subtypes of migraine. International Journal of Epidemiology 1995; 24:612-618.
* Schuh-Hofer S, Reuter U, Kinze S, Einhäupl KM, Amold G. Treatment of acute cluster headache attacks with 20 mg sumatriptan nasal spray-an open pilot study. J Neurol 2002; 249: 94-99.
* Shioe K, Ichimiya T, Sahara T, Takano A, Suite Y, Yasuno F, Hirano M, Shinohara M, Kagami M, Okubo Y, Nankai M, Kanba S. No association between genotype of the promoter region of serotonin transporter gene and serotonin transporter binding in human brain measured by PET. Synapse 2003; 48:184-188.
* Silberstein SD. Methysergide. Cephalalgia 1998; 18: 421-435.
* Silberstein SD. Practice parameter: Evidence-based guidelines for migraine headache (an evidence-based review)- Neurology 2000; 55: 754-763.
* Srikiatkhachorn A, Anthony M. Platelet serotonin in patients with analgesic-induced headache. Cephalalgia 1996; 16: 423-6.
* Tauscher J, Pirker W, de Zwaan M, Asenbaum S, Brucke T, Kasper S: In vivo visualization of serotonin transporters in the human brain during fluoxetine treatment. Eur Neuropsychopharmacol 1999; 9: 177-9.
* Törk I. Anatomy of the Serotonergic System. Annals of the New York Academy of Science 1990; 600: 9-34.
* Weiller C, May A, Limmroth V, Jüptner M, Kaube H, Schayck RV, Coenen HH. Diener HC. Brain stem activation in spontaneous human migraine attacks. Nature medicine 1995; 1: 658-660.
* Willeit M, Praschak-Rieder N, Neumeister A, Pirker W, Asenbaum S, Vitouch O et al., [123I]-ß-SPECT imaging shows reduced brain serotonin transporter availability in drug-free depressed patients with seasonal affective disorders. Biol Psychiatry 2000; 47: 482-489.
* Yilmaz M, Erdal ME, Herken H, Cataloluk O, Barlas Ö, Bayazit YA- Significance of serotonin transporter gene polymorphism in migraine. Journal of Neurol Sci 2001; 186: 27-30.

## Patentansprüche

1. Verwendung von spezifisch an das Serotonin-Transport-Protein (SERT) bindenden Radiopharmaka zur Herstellung eines Diagnostikums zur Untersuchung von Kopfschmerzpatienten.

2. Verwendung nach Anspruch 1, wobei das ausgewählte Radiopharmakon ¹²³I-ADAM für die Hirn-SPECT-Szintigraphie ist.

3. Verwendung nach Anspruch 1 oder 2, wobei das Radiopharmakon ein herkömmliches PET-Radiopharmakon, ausgewählt aus ¹⁵O-H₂O-Liganden, ¹¹C-McN 5652, (S)-([18F]fluoromethyl)-(+)-McN5652, ¹¹C-DASB und ¹¹C-DAPP ist.

4. Verwendung nach Anspruch 1 oder 2, wobei das Radiopharmakon ein herkömmliches SPECT-Radiopharmakon, ausgewählt aus ¹²³I--ADAM ¹²³I--IDAM, ¹²³I--ODAM, ¹²³Iß-CIT und ¹²³I-nor-ß-CIT ist.

5. Verwendung nach einem der Ansprüche 1 bis 4, wobei das Radiopharmakon zur Messung der Verfügbarkeit des SERT in der pontomesencephalen Region des Gehirns, die die Region des rostralen Raphe-Kemkomplexes umfasst, verwendet wird.

6. Verwendung nach einem der Ansprüche 1 bis 6, wobei das Radiopharmakon in der Therapieverlaufskontrolle der Migräne, des Clusterkopfschmerzes oder des arzneimittelinduzierten Kopfschmerzes verwendet wird.

7. In vitro Verfahren zum Screening auf Substanzen, die die Bindung eines spezifisch an das Serotonin-Transport-Protein (SERT) bindenden Radiopharmakons moduliert, umfassend die Schritte von:
a) zur Verfügung stellen eines in vitro-Systems, welches den SERT und das SERT-spezifische Radiopharmakon umfaßt,
b) zur Verfügung stellen einer Kandidaten-Substanz,
c) in Kontakt bringen von SERT und SERT-spezifischem Radiopharmakon zusammen mit der Kandidaten-Substanz und ohne die Kandidaten-Substanz, und
d) Messen der Bindung zwischen SERT und SERT-spezifischem Radiopharmakon, wobei ein Unterschied in der Bindung mit der Kandidaten-Substanz und ohne die Kandidaten-Substanz eine Substanz identifiziert, die die Bindung eines spezifisch an das Serotonin-Transport-Protein (SERT) bindenden Radiopharmakons modifiziert.

8. Verfahren nach Anspruch 7, wobei der SERT des in vitro-Systems aus der pontomesencephalen Region des Gehirns und insbesondere aus den serotonergen Raphe-Kernen stammt.

9. Verfahren nach Anspruch 7 oder 8, wobei das Radiopharmakon ausgewählt ist aus herkömmlichen PET- oder SPECT-Radiopharmaka.

10. Verfahren nach einem der Ansprüche 7 bis 9, wobei die Modulation eine Inhibierung oder Erhöhung der Bindung des Radiopharmakons an den SERT ist.

11. Verfahren zur Herstellung eines Pharmazeutikums zur Behandlung von Kopfschmerzen, umfassend ein Verfahren nach einem der Ansprüche 7 bis 9 und Mischen der identifizierten Substanz mit einem geeigneten pharmazeutischen Träger.
